# EUROPEAN PATENT APPLICATION

(11) **EP 4 206 214 A1**
(43) Date of publication of application: **05.07.2023**
(21) Application number: 21861775.1
(22) Date of filing: 31.08.2021
(51) Int. Cl.: C07J 9/00, C07J 53/00, A23L 33/11

(54) **METHOD FOR PRODUCING PHYTOSTEROL STARTING MATERIAL**

(30) Priority: 31.08.2020 JP 2020145490
(71) Applicant: Morinaga Milk Industry Co., Ltd., Minato-ku Tokyo 108-8384 (JP)
(72) Inventor: SHIINA, Tomohiro, Zama-shi, Kanagawa 252-8583 (JP); MOTOYOSHI, Tomomi, Zama-shi, Kanagawa 252-8583 (JP); OZAWA, Tomohito, Zama-shi, Kanagawa 252-8583 (JP)
(74) Representative: Vossius & Partner Patentanwälte Rechtsanwälte mbB
(86) International application number: PCT/JP2021/031929
(87) International publication number: WO 2022/045354

(57) **Abstract**

A problem of the invention is to provide development of a simpler and more efficient method for producing a phytosterol starting material. The invention provides a method for producing a phytosterol starting material which includes the step (1) and the step (2) below: a step (1) which is a step of squeezing aloe mesophyll and thus obtaining a squeezing residue; and a step (2) which is a step of drying the squeezing residue obtained in the step (1) and thus obtaining a dried squeezing residue.

## Description

### Technical Field

The present invention relates to a method for producing a phytosterol starting material and the like.

### Background Art

Aloe is a kind of succulent plant including *Aloe vera* (*Aloe barbadensis* Miller), *Aloe arborescens* (*Aloe arborescens* Miller var. *natalensis* Berger) and the like and is empirically known to have various effects. Recently, the functionality of aloe has been drawing attention, and applications for functional foods, supplements, medicines, cosmetics and the like have been examined.

Aloe is also known to contain a tiny amount of phytosterols. It has been found that phytosterols contained in aloe exhibit a hyperglycemia-improving effect and the like (PTL 1). Various techniques are under development for producing an extract in which phytosterols contained in aloe, which are such functional components, are concentrated or for purifying phytosterols.

PTL 1 discloses a method for producing an *Aloe vera* extract containing phytosterols contained in natural *Aloe vera* by supercritical extraction from powdery *Aloe vera* mesophyll prepared by drying a mesophyll part excluding the leaf skin of *Aloe vera.* PTL 2 discloses a method for recovering phytosterols from rapeseed oil and soybean oil using methanol and a method for separating phytosterols by immersing crude phytosterols in an organic solvent and then separating the organic solvent. Although phytosterols contained in aloe can be concentrated by the methods, development of a simpler and more efficient method for producing a phytosterol starting material with shortened treatment time has been desired.

### Citation List

### Patent Literature

PTL 1: WO2007/060911
PTL 2: JP-T-2002-542161 (the term "JP-T" as used herein means a published Japanese translation of a PCT patent application.)

### Summary of Invention

### Technical Problem

Considering the above circumstances, a problem is to provide development of a simpler and more efficient method for producing a phytosterol starting material.

### Solution to Problem

The inventors have conducted intensive investigation for the purpose of obtaining a simpler and more efficient method for producing a phytosterol starting material. The inventors have found that the drying treatment time during the production of a phytosterol starting material can be shortened by squeezing aloe mesophyll to separate it into squeezed juice and a squeezing residue and using the dried squeezing residue as a phytosterol starting material. Moreover, the inventors have found that a phytosterol starting material composed of such a dried squeezing residue has a higher phytosterol content than phytosterol starting materials such as dried aloe mesophyll and dried squeezed juice. The invention has been completed based on the findings.

That is, the invention is as follows.
[1] A method for producing a phytosterol starting material including the step (1) and the step (2) below:
   a step (1) which is a step of squeezing aloe mesophyll and thus obtaining a squeezing residue; and
   a step (2) which is a step of drying the squeezing residue obtained in the step (1) and thus obtaining a dried squeezing residue.
[2] The method described in [1], wherein the ratio of the phytosterol amount in the squeezing residue to the phytosterol amount in the aloe mesophyll before the squeezing in the step (1) is 70% or more.
[3] The method described in [1] or [2], wherein the squeezing is conducted with a screw squeezer.
[4] The method described in any of [1] to [3], wherein the drying is conducted by freeze-drying, hot-air drying, indirect heat drying, blast drying, dehumidifying air drying or vacuum/reduced pressure drying.
[5] The method described in any of [1] to [4], wherein the phytosterol is one, two or more compounds selected from the group consisting of lophenol compounds and cyclolanostane compounds.
[6] The method described in any of [1] to [5] further including
   a step (3) which is a step of extracting a phytosterol-containing fraction from the dried squeezing residue obtained in the step (2).
[7] The method described in [6], wherein the extraction is conducted by a supercritical extraction method.
[8] The method described in any of [1] to [7], wherein the phytosterol starting material is a starting material for producing a phytosterol-containing food.

### Advantageous Effects of Invention

According to the invention, a method for producing a phytosterol starting material in which the drying treatment time during the production of the phytosterol starting material is shortened and which is simpler and more efficient than the conventional methods is provided.

Moreover, by the production method provided by the invention, a phytosterol starting material having a high phytosterol content is provided. When a functional food is developed using a plant such as aloe, the non-active ingredient content of aloe should be considered for considering the intake which guarantees the functionality. That is, a material which is suitable for developing a functional food using aloe can be provided by the invention, and it is believed that the application of aloe to foods and the like is promoted.

### Brief Description of Drawings

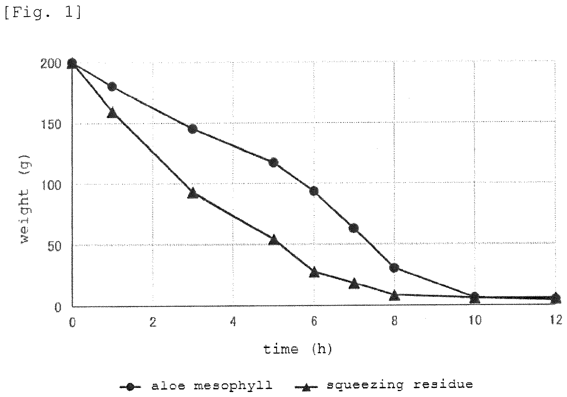

[Fig. 1] Fig. 1 is a figure showing the results of a drying test of aloe mesophyll (after cutting) and a squeezing residue.

### Description of Embodiments

Embodiments of the invention are explained below. In this regard, however, the invention is not limited to the following preferable embodiments, and changes can be freely made within the scope of the invention. The percentages (%) in the present description are weight % unless otherwise specified.

### <Production Method of Phytosterol Starting Material>

In an embodiment, the invention relates to a method for producing a phytosterol starting material including the step (1) and the step (2) below (sometimes called "the method for producing a phytosterol starting material of the invention" below):
a step (1) which is a step of squeezing aloe mesophyll and thus obtaining a squeezing residue; and
a step (2) which is a step of drying the squeezing residue obtained in the step (1) and thus obtaining a dried squeezing residue.

As explained above, while dried aloe mesophyll prepared by cutting and drying aloe mesophyll has been conventionally used as a phytosterol starting material, the inventors have attempted to use a squeezing residue obtained by squeezing aloe mesophyll to separate it into a squeezed juice and a squeezing residue and drying as a phytosterol starting material. The inventors have found that the drying treatment time during the production of a phytosterol starting material can be shortened by such a method. Moreover, it has not been clear which of the squeezed juice and the squeezing residue obtained by squeezing aloe mesophyll contains phytosterols, but the inventors have focused on the squeezing residue and obtained a phytosterol starting material having a high phytosterol content composed of a dried squeezing residue. The invention has been thus completed.

In the present description, the "phytosterol starting material" is a material containing a phytosterol and is a starting material for producing a phytosterol through extraction, purification or the like, for producing a composition containing a high amount of a phytosterol, and the like. The final form of the "phytosterol starting material" is not particularly limited. For example, those in a solid form (a dried product and powder thereof), a semi-solid form and the like are included in the "phytosterol starting material" in the present description. An "aloe extract" in a liquid form or the like which is obtained by extraction treatment of a phytosterol from a phytosterol starting material and in which the phytosterol is concentrated is also included in the "phytosterol starting material" in the present description. That is, the "phytosterol starting material" in the present description includes all the forms of liquid, solid (a dried product and powder thereof), semi-solid and the like.

In another embodiment, the invention relates to a method for producing a phytosterol-containing composition. The invention in the embodiment relates to a method for producing a phytosterol-containing composition including the step (1) and the step (2) below:
a step (1) which is a step of squeezing aloe mesophyll and thus obtaining a squeezing residue; and
a step (2) which is a step of drying the squeezing residue obtained in the step (1) and thus obtaining a dried squeezing residue as a phytosterol-containing composition.

In another embodiment, the invention relates to a method for treating aloe mesophyll.

That is, the invention in the embodiment relates to a method for treating aloe mesophyll including the step (1) and the step (2) below:
a step (1) which is a step of squeezing aloe mesophyll and thus obtaining a squeezing residue; and
a step (2) which is a step of drying the squeezing residue obtained in the step (1) and thus obtaining a dried squeezing residue.

### <Step (1): Step of Squeezing Aloe Mesophyll and Obtaining Squeezing Residue>

The step of squeezing aloe mesophyll and thus obtaining a squeezing residue is a step of subjecting aloe mesophyll to squeezing treatment, removing squeezed juice and thus obtaining a squeezing residue.

### <<Aloe Mesophyll>>

In the present description, the "aloe mesophyll" is mesophyll of a plant belonging to the genus *Aloe* of the family Liliaceae. The plant belonging to the genus *Aloe* is not limited, but examples thereof include *Aloe vera* (*Aloe barbadensis* Miller), *Aloe arborescens* (*Aloe arborescens* Miller var. *natalensis* Berger), *Aloe ferox* (*Aloe ferox* Miller) and the like. Of these, *Aloe vera* and *Aloe arborescens* are preferably used.

When a leaf of aloe is sliced crosswise, the external wall of the epidermis covered with a thick cuticle layer appears. Mesophyll which is differentiated into chlorenchyma tissue cells and cells with a thin cell wall known as parenchyma is found below the epidermis. The parenchyma cells store mucilage-like transparent jelly. The vascular bundle having internal bundle sheath cells contains yellow latex having the properties of a laxative and is between two large cells. That is, aloe has two kinds of major liquid sources, namely yellow latex (exudate) and transparent gel (mucilage). The transparent gel (mucilage) is called aloe mesophyll (aloe gel). In this manner, a leaf of aloe can be divided into three parts of (A) yellow latex, (B) aloe mesophyll, and (C) leaf skin composed of an outer rind, a tip, a base and spikes.

The "aloe mesophyll" in the invention includes (B) aloe mesophyll (aloe gel). The aloe mesophyll used in the invention may include (A) yellow latex and (C) leaf skin in addition to (B) aloe mesophyll but preferably does not include (A) yellow latex or (C) leaf skin.

The aloe mesophyll can be prepared by a normal method. For example, the aloe mesophyll can be prepared through steps of peeling the leaf skin off an aloe leaf, further removing the yellow latex by washing and recovering the mesophyll of aloe.

A commercial product of aloe mesophyll may also be used.

### <<Squeezing of Aloe Mesophyll>>

By subjecting aloe mesophyll to squeezing treatment and removing the squeezed juice, a squeezing residue is obtained. The squeezer used for the squeezing treatment in the step (1) in the production method of the invention is not particularly limited as long as the squeezer can press the aloe mesophyll and discharge the squeezed juice.

In the squeezing treatment in the step (1), the ratio of the phytosterol amount in the squeezing residue to 100% of the phytosterol amount in the aloe mesophyll before the squeezing in the step (1) is preferably 70% or more, 80% or more, or 90% or more. The ratio of the phytosterol amount in the squeezing residue to 100% of the phytosterol amount in the aloe mesophyll before the squeezing can be determined specifically as follows. A squeezing residue is obtained by subjecting aloe mesophyll to squeezing treatment and removing the squeezed juice. The ratio can be determined as (the total phytosterol amount contained in the obtained squeezing residue/the total phytosterol amount in the aloe mesophyll before the squeezing) × 100. Moreover, in the method for producing a phytosterol starting material of the invention, a squeezer which can achieve such a value can be preferably used.

Examples of the squeezer used for the squeezing treatment in the step (1) include: a hydraulic squeezer in which pressure is applied to the starting material put into a squeezer tank with a slide to squeeze the juice; a screw squeezer which is composed of an outer cylinder having a filtration function structure that passes liquid only without passing solid particles and a screw and in which the starting material is squeezed by compressing and forcing out the starting material that is continuously supplied from the screw base to the screw end side through the rotation of the screw, squeezing the juice contained in the starting material during the compression process and discharging the squeezed juice outside from the filtration function structure of the outer cylinder; and the like. One type thereof or a combination of two or more types thereof may be used.

Of these, a screw squeezer is suitable, for example, because a relatively high phytosterol content of the squeezing residue is achieved and because the drying time of the squeezing residue can be made relatively short. As the screw squeezer, for example, a screw squeezer (type OMST-90B) manufactured by OHMICHI Co., Ltd. can be used. The squeezing conditions can be appropriately determined depending on the sample amount, the aimed water content and the like. For squeezing with a screw squeezer, for example, the conditions of a screw rotation speed of 18 to 36 rpm and a processing amount of around 30 to 50 kg/hr can be used.

The water content is preferably removed in such a manner that the water content of the aloe mesophyll becomes, for example, 99.5% (weight % concentration) or less, 99.0% (weight % concentration) or less, or 98.5% (weight % concentration) or less after the completion of the step (1).

### <Step (2): Step of Drying Squeezing Residue and Obtaining Dried Squeezing Residue>

The step of drying the squeezing residue and thus obtaining a dried squeezing residue is a step of removing the water content by drying the squeezing residue obtained in the step (1) and thus obtaining a dried squeezing residue. The dried squeezing residue is used as a phytosterol starting material.

As explained above, in the conventional methods, dried aloe mesophyll prepared by cutting and drying aloe mesophyll is used as a phytosterol starting material. On the other hand, in the method for producing a phytosterol starting material of the invention, a dried squeezing residue prepared by drying a squeezing residue is used as a phytosterol starting material, and the time of the drying step can be shortened.

Regarding the time required for drying the squeezing residue in the method for producing a phytosterol starting material of the invention, the time required until the weight of the starting material is reduced to 50% may be, for example, 0.9 times the time required for drying aloe mesophyll in the conventional methods or less, 0.7 times the time or less, or 0.6 times the time or less.

### <<Drying of Squeezing Residue>>

The drying method used in the step (2) in the production method of the invention is not particularly limited as long as the water content can be removed from the squeezing residue, and examples thereof include freeze-drying, hot-air drying, indirect heat drying, blast drying, dehumidifying air drying, vacuum/reduced pressure drying and the like. One type thereof or a combination of two or more types thereof may be used.

Of these, freeze-drying is suitable in view of the maintenance of the aroma, the high drying degree or the like, and hot-air drying is suitable in view of the cost, the efficiency or the like. Freeze-drying can be conducted, for example, using a freeze dryer (type FZ-6CS) manufactured by Labconco. Hot-air drying can be conducted, for example, using a hot-air dryer (type DK63) manufactured by Yamato Scientific Co., Ltd. The drying conditions can be appropriately determined depending on the sample amount, the aimed drying degree and the like. Freeze-drying can be conducted, for example, under the conditions of a drying plate temperature of 20 to 30°C, a vacuum degree of 0.0015 to 0.0998 mmHg for around 24 to 36 hours. Hot-air drying can be conducted, for example, under the conditions of a hot-air temperature of 50 to 70°C for around 6 to 12 hours.

The water content is preferably removed in such a manner that the water content of the dried squeezing residue becomes, for example, 20% (weight % concentration) or less, 15% (weight % concentration), or less or 10% (weight % concentration) or less after the completion of the step (2). The dried squeezing residue after the removal of the water content is generally in a rough powder form.

The phytosterol is preferably one, two or more compounds selected from the group consisting of the lophenol compounds and the cyclolanostane compounds described below.

The phytosterol is more preferably Aloesterol (registered trademark of MORINAGA MILK INDUSTRY CO., LTD.). Aloesterol is a generic term for five phytosterols contained in the mesophyll part of *Aloe vera* (4-methylcholest-7-en-3-ol, 4-methylergost-7-en-3-ol, 4-methylstigmast-7-en-3-ol, 9,19-cyclolanostan-3-ol and 24-methylene-9,19-cyclolanostan-3-ol).

The lophenol compounds are represented by the general formula (1) below.

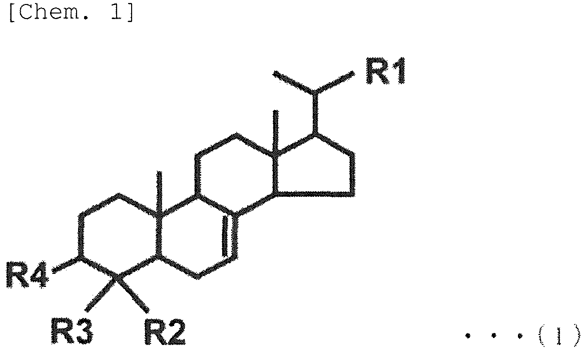

In the general formula (1), R1 is a linear or branched alkyl group having 5 to 16 carbon atoms or an alkenyl group including one or two double bonds. The alkyl group or the alkenyl group may be a substituted alkyl group or a substituted alkenyl group in which one or two hydrogen atoms are substituted with a hydroxy group and/or a carbonyl group.

R2 and R3 are each independently a hydrogen atom or an alkyl group having 1 to 3 carbon atoms. The alkyl group having 1 to 3 carbon atoms is preferably a methyl group, an ethyl group or the like, particularly preferably a methyl group. The alkyl group having 1 to 3 carbon atoms may be a substituted alkyl group in which at least one hydrogen atom is substituted with a hydroxy group and/or a carbonyl group and is specifically any of the groups represented by the formulae below.

-CH₂-OH

-CH₂-COOH

-CH₂-CH₂-OH

-CH₂-CH₂-COOH

-CH(OH)-CH₃

-CH(COOH)-CH₃

R4 is a group forming C=O with the carbon atom forming the ring, a hydroxy group or -OCOCH₃.

In the general formula (1), R1 is preferably any of the groups represented by the formulae below.

-CH₂-CH₂-CH(-CH₂-CH₃)-CH(CH₃)₂

-CH₂-CH₂-CH=C(CH₃)₂

-CH₂-CH=C(CH₃)-CH(CH₃)₂

-CH₂-CH₂-C(=CH-CH₃)-CH(CH₃)₂

-CH₂-CH₂-C(Ra)=C(CH₃)Rb

(Here, Ra and Rb are independently a hydrogen atom, a hydroxy group or a methyl group.)

-CH₂-CH₂-CH(Rc)-CH(CH₃)Rd

(Here, Rc and Rd are independently a hydrogen atom, a hydroxy group or a methyl group.)

In the general formula (1), it is preferable that one of R2 and R3 is a hydrogen atom and that the other is a methyl group, and R4 is preferably a hydroxy group.

The lophenol compounds are preferably 4-methylcholest-7-en-3-ol, 4-methylergost-7-en-3-ol and 4-methylstigmast-7-en-3-ol. The compounds have the structures represented by the formulae below, respectively.

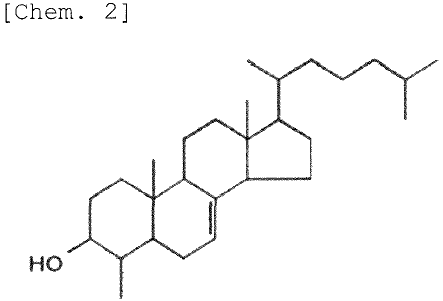

### 4-Methylcholest-7-en-3-ol

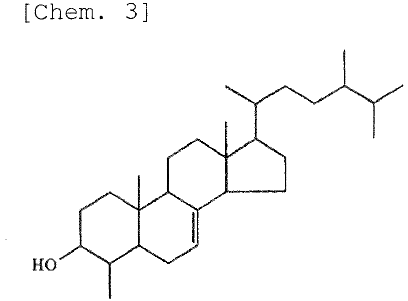

### 4-Methylergost-7-en-3-ol

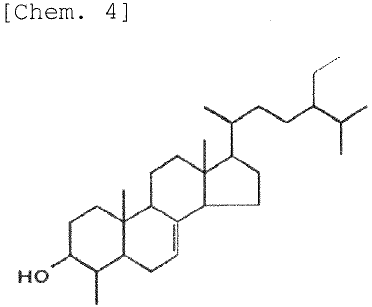

### 4-Methylstigmast-7-en-3-ol

The cyclolanostane compounds are represented by the general formula (2) below.

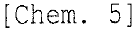

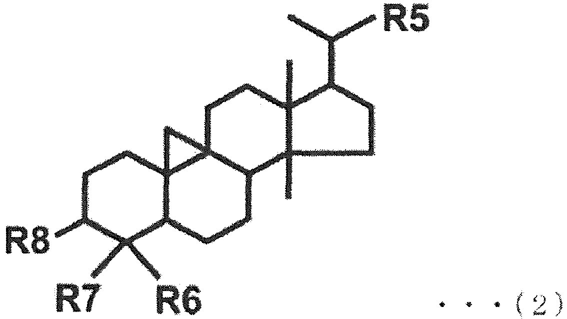

In the general formula (2), R5 is a linear or branched alkyl group having 6 to 8 carbon atoms or an alkenyl group including one or two double bonds. The alkyl group or the alkenyl group may be a substituted alkyl group or a substituted alkenyl group in which one or two hydrogen atoms are substituted with a hydroxy group and/or a carbonyl group.

R6 and R7 are each independently a hydrogen atom or a methyl group. R8 is a group forming C=O with the carbon atom forming the ring or any of the groups below.

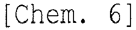

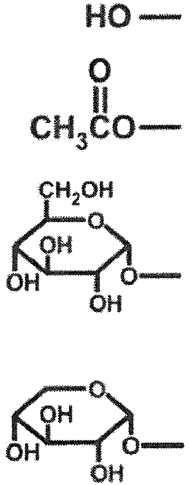

In the general formula (2), R5 is preferably any of the groups represented by the formulae below.

-CH₂-CH₂-CH₂-CH(CH₃)₂

-CH₂-CH₂-CHRe-C(CH₃)₂Rf

(Here, Re is a hydrogen atom, a hydroxy group or a methyl group, and Rf is a hydrogen atom or a hydroxy group.)

-CH₂-CH₂-CH(CH₂-CH₃₎-CH(CH₃)₂

-CH₂-CH₂-CHRg-C(CH₃)=CH₂

(Here, Rg is a hydrogen atom, a hydroxy group or a methyl group.)

-CH₂-CH₂-C(=O)-C(CH₃)=CH₂

-CH₂-CH₂-C(=CH₂)-CH(CH₃)₂

-CH₂-CH₂-CH=C(CH₃)₂

-CH₂-CH=C(CH₃)-CH(CH₃)₂

-CH₂-CH₂-C(=CH-CH₃)-CH(CH₃)₂

In the general formula (2), it is preferable that one of R6 and R7 is a hydrogen atom and that the other is a methyl group, and R8 is preferably a hydroxy group.

The cyclolanostane compounds are preferably 9,19-cyclolanostan-3-ol and 24-methylene-9,19-cyclolanostan-3-ol. The compounds have the structures represented by the formulae below, respectively.

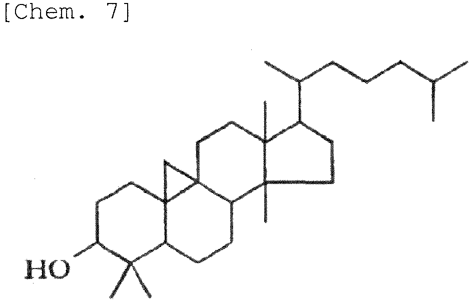

### 9,19-Cyclolanostan-3-ol

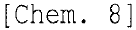

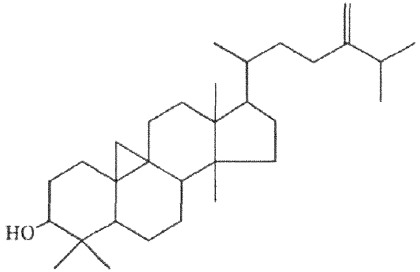

### 24-Methylene-9,19-cyclolanostan-3-ol

In another embodiment, the invention relates to a method for producing a phytosterol starting material further including a step (3) which is a step of extracting a phytosterol-containing fraction from the dried squeezing residue obtained in the step (2) in addition to the steps (1) and (2).

### <Step (3): Step of Extracting Phytosterol-Containing Fraction from Dried Squeezing Residue>

The step (3) is a step of extracting a phytosterol-containing fraction from the dried squeezing residue obtained in the step (2). The extract extracted from the dried squeezing residue is an aloe extract containing a phytosterol-containing fraction. As described above, in the present description, the aloe extract is also a phytosterol starting material.

<<Extraction of Phytosterol-Containing Fraction>>

The extraction method used in the step (3) in the production method of the invention is not particularly limited as long as a phytosterol-containing fraction can be extracted from the dried squeezing residue, and the extraction method can be conducted by a normal method. Examples thereof include a supercritical extraction method, an organic solvent extraction method and the like. Of these, a supercritical extraction method is suitable in view of the safety of the operation and the product or the like. The supercritical extraction method can be conducted, for example, by the method below.

The extraction solvent used for the supercritical extraction is not particularly restricted but is preferably a solvent which can extract components that cause the fishy odor peculiar to aloe, such as butyric acid, 2,4-heptadienal and 2-ethylhexanol, especially 2,4-heptadienal. For example, carbon dioxide, supercritical propane, supercritical ethylene, supercritical 1,1,1,2-tetrafluoroethane and the like can be used.

When the phytosterol starting material produced by the production method of the invention is used as a component of a food, a drink, a medicine, a cosmetic, feed or the like, carbon dioxide is preferably used as the extraction solvent from the viewpoint of emphasizing safety.

Butyric acid is a colorless liquid having a rotten butter-like unpleasant acid odor. 2,4-Heptadienal has a pungent green smell and is a colorless liquid having a grassy smell. 2-Ethylhexanol is a colorless liquid having a roselike floral aroma which can be an unfavorable odor depending on the amount.

The extraction temperature can be appropriately selected depending on the kind of the extraction solvent and the other conditions and can be, for example, 28 to 120°C as a rough standard. To extract the odor components peculiar to aloe sufficiently or to obtain the physical properties suitable for crushing described below, the extraction temperature is preferably 31 to 80°C, further preferably 50 to 69°C, more preferably 50 to 59°C as a rough standard.

The pressure can be appropriately selected depending on the kind of the extraction solvent and the other conditions and can be, for example, 5.5 to 60 MPa as a rough standard. To extract the odor components peculiar to aloe sufficiently or to obtain the physical properties suitable for crushing described below, the pressure is preferably 7 to 60 MPa, further preferably 15 to 60 MPa, more preferably 15 to 24 MPa as a rough standard.

The extraction time can be appropriately selected depending on the kind of the extraction solvent and the other conditions and can be, for example, 30 seconds to 7 hours as a rough standard.

The extraction conditions are, for example, the conditions of a) to d) below.
a) The extraction solvent is carbon dioxide.
b) The extraction temperature is 31 to 80°C.
c) The pressure is 7 to 60 MPa.
d) The extraction time is 30 seconds to 7 hours.

In the supercritical extraction, an entrainer such as ethanol can also be used to extract the odor components peculiar to aloe sufficiently.

After conducting the steps (1) and (2) or the steps (1) to (3) in the production method of the invention, a step required for preparing the phytosterol starting material, such as a crushing step and a purification step, may further be conducted according to the need. The steps can be conducted by normal methods.

<<Phytosterol-Containing Food>>

In another embodiment, the invention relates to a method for producing a phytosterol starting material in which the phytosterol starting material is a starting material for producing a phytosterol-containing food.

The phytosterol-containing food is not particularly restricted as long as the effects of the phytosterol are not impaired. The food may include a drink. The food or the drink can be in any form such as a solid, a liquid and a semi-solid. Such foods include health foods, functional foods, supplements, enteral nutrition products, foods for special dietary uses, foods with health claims, foods for specified health uses, foods with nutrient function claims and the like. The phytosterol starting material of the invention contains a higher amount of a phytosterol than that of a phytosterol starting material obtained by the conventional production method (a method of drying aloe mesophyll without squeezing). As a result, the phytosterol starting material can be suitably applied to, for example, health foods, functional foods, supplements, enteral nutrition products, foods for special dietary uses, foods with health claims, foods for specified health uses, foods with nutrient function claims and the like.

The phytosterol-containing food can be produced based on a known method for producing a food except that a phytosterol starting material produced by the production method of the invention is added.

The amount of the phytosterol starting material of the invention contained in the food is not particularly restricted as long as the effects of the phytosterol are obtained, but the amount is preferably 0.01 to 50 weight%, particularly preferably 0.01 to 20 weight%.

### Examples

The invention is explained further specifically below with Examples, but the invention is not limited to the Examples unless the gist is exceeded. In the Examples of the present description, *Aloe vera* was used as aloe.

### <Example 1> Production of Squeezing Residue and Dried Squeezing Residue and Measurement of Aloesterol Content

The leaf skin of 10 kg of aloe was peeled off, and the mesophyll part was collected. The collected mesophyll part was squeezed with a screw squeezer (a screw diameter of 9.5 cm; type OMST-90B; manufactured by OHMICHI Co., Ltd.) at a screw rotation speed of 36 rpm and a processing amount of 30 kg/hr, and 7.03 kg of squeezed juice (juice) and 2.18 kg of a squeezing residue (squeezing remains) were thus obtained. One kilogram of the obtained squeezing residue was freeze-dried for 36 hours with a freeze dryer (type FZ-6CS; manufactured by Labconco) under the conditions of a drying plate temperature of 25°C and a vacuum degree of 0.0998 mmHg or less, and 35.3 g of a dried squeezing residue was thus prepared.

As a Comparative Example, 1 kg of the squeezed juice obtained in the above step was freeze-dried for 36 hours with a freeze dryer under the conditions of a drying plate temperature of 25°C and a vacuum degree of 0.0998 mmHg or less, and 4.6 g of a dried squeezed juice was thus prepared. Moreover, the leaf skin of 1.0 kg of aloe was peeled off, and the mesophyll part was collected. The collected mesophyll part was cut into dices and freeze-dried for 36 hours with a freeze dryer under the conditions of a drying plate temperature of 25°C and a vacuum degree of 0.0998 mmHg or less, and 7.7 g of dried aloe mesophyll was thus prepared.

Regarding the squeezing residue before drying, the dried squeezing residue, the squeezed juice before drying, the dried squeezed juice, the aloe mesophyll before drying and the dried aloe mesophyll, the amounts of the contained Aloesterols (9,19-cyclolanostan-3-ol and 24-methylene-9,19-cyclolanostan-3-ol, which are cyclolanostane compounds, and 4-methylcholest-7-en-3-ol, 4-methylergost-7-en-3-ol and 4-methylstigmast-7-en-3-ol, which are lophenol compounds) were measured using LC/MS/MS (type Agilent 6460; manufactured by Agilent Technologies, Inc.) using brassicasterol (manufactured by FUJIFILM Wako Pure Chemical Corporation) as an internal standard.

The results are shown in Table 1.

### [Table 1]

**Table 1**

| Squeezing [OHMICHI Co., Ltd. (OMST-90B)] | | Recovery Rate of Squeezing Residue and Squeezed Juice | Mass Balance of Sterols | Aloesterol Content (µg/kg) (Per 1 kg after Separation) | Aloesterol Content (µg/kg) (Per 1 kg Starting Material) | Aloesterol Content (µg/g) (Per 1 g Dried Powder) |
|---|---|---|---|---|---|---|
| Before Squeezing | Aloe Mesophyll (Starting Material) | 100% | 100% | 483.5 | 483.5 | 62.4 |
| After Squeezing | Squeezing Residue | 22% | 103% | 2283.5 | 498.0 | 141.1 |
| | Squeezed Juice | 70% | 14% | 93.6 | 65.7 | 17.8 |

Through squeezing, the aloe mesophyll could be separated into a squeezing residue and squeezed juice (squeezing residue:squeezed juice≈1:4). Moreover, as shown in Table 1, it was found that most of the Aloesterols remained in the squeezing residue (the mass balance of sterols was 1030). It was found that, as a result, the squeezing residue contained about five times more Aloesterols than the aloe mesophyll. It was also found that the dried squeezing residue contained two or more times of the Aloesterols compared to the dried aloe mesophyll.

From the above results, it was found that a dried squeezing residue obtained by drying a squeezing residue can be suitably used as a phytosterol starting material.

### <Example 2> Measurement of Water Evaporation Efficiencies of Aloe Mesophyll and Squeezing Residue

The aloe mesophyll (after cutting) and the squeezing residue obtained in Example 1 each in an amount of 200 g were placed in a shelf dryer (hot-air circulation thermostatic dryer; type DK-63; manufactured by Yamato Scientific Co., Ltd.)) and treated at 60°C for 12 hours. During the drying, the sample weights were measured over time.

The results are shown in Fig. 1. As shown in Fig. 1, it was found that water evaporated more easily from the squeezing residue than from the aloe mesophyll.

From the above results, it was found that the drying treatment time is shortened in the method for producing a phytosterol starting material of the invention as compared to that of the conventional method.

### Industrial Applicability

The invention is useful in the fields of foods, health foods, functional foods, supplements and the like.

## Claims

1. A method for producing a phytosterol starting material including the step (1) and the step (2) below:
a step (1) which is a step of squeezing aloe mesophyll and thus obtaining a squeezing residue; and
a step (2) which is a step of drying the squeezing residue obtained in the step (1) and thus obtaining a dried squeezing residue.

2. The method according to claim 1, wherein the ratio of the phytosterol amount in the squeezing residue to the phytosterol amount in the aloe mesophyll before the squeezing in the step (1) is 70% or more.

3. The method according to claim 1 or 2, wherein the squeezing is conducted with a screw squeezer.

4. The method according to any one of claims 1 to 3, wherein the drying is conducted by freeze-drying, hot-air drying, indirect heat drying, blast drying, dehumidifying air drying or vacuum/reduced pressure drying.

5. The method according to any one of claims 1 to 4, wherein the phytosterol is one, two or more compounds selected from the group consisting of lophenol compounds and cyclolanostane compounds.

6. The method according to any one of claims 1 to 5 further including
a step (3) which is a step of extracting a phytosterol-containing fraction from the dried squeezing residue obtained in the step (2).

7. The method according to claim 6, wherein the extraction is conducted by a supercritical extraction method.

8. The method according to any one of claims 1 to 7, wherein the phytosterol starting material is a starting material for producing a phytosterol-containing food.
